# EUROPEAN PATENT SPECIFICATION

(11) **EP 1 496 988 B1**
(45) Date of publication and mention of the grant of the patent: **01.07.2009**
(21) Application number: 03721694.2
(22) Date of filing: 16.04.2003
(51) Int. Cl.: A61N 1/32

(54) **VASCULAR ELECTRODE SYSTEM FOR ELECTRICAL STIMULATION FOR THROMBOLYTIC THERAPY**
GEFÄSSELEKTRODENSYSTEM FÜR DIE ELEKTRISCHE STIMULATION ZUR THROMBOLYTISCHEN THERAPIE
SYSTEME D'ELECTRODES VASCULAIRES PROUR STIMULATION ELECTRIQUE POUR TRAITEMENT THROMBOLYTIQUE

(30) Priority: 16.04.2002 US 373029 P; 15.04.2003 US 414382
(43) Date of publication of application: 19.01.2005
(73) Proprietor: MEDTRONIC, INC., Minneapolis MN 55432-5604 (US)
(72) Inventor: SOYKAN, Orhan, Shoreview, MN 55126 (US); DENO, Curtis, D., Andover, MN 55304 (US); TRESCONY, Paul, V., Champlin, MN 55316 (US); DONOVAN, Maura, G., St. Paul, MN 55105 (US); WILLIAMS, Terrell, M., Brooklyn Park, MN 55444 (US); ROBINSON, Timothy, H., Savage, MN 55378 (US)
(74) Representative: Hughes, Andrea Michelle
(86) International application number: PCT/US2003/011677
(87) International publication number: WO 2003/089054

(56) References cited:
- WO-A-00/27466
- WO-A-01/72234
- WO-A-02/26314
- GB-A- 1 296 550
- US-A- 5 351 394
- US-A1- 2002 010 492
- KANOKWAN TILOKSKULCHAI, ET.AL.: "The effects of electrical stimulation on the level of tissue plaminogen activator, plasmiongen activator inhibitor and the fibrinolytic activity in rats after permanent unilateral common carotid artery occlusion" MAHIDOL UNIVERSITY ANNUAL RESEARCH ABSTRACTS 2000 , [Online] vol. 27, 2000, page 309 XP002249723 Retrieved from the Internet: <URL:http://www.mahidol.ac.th/abstracts/an nual1999/> [retrieved on 2003-07-31]
- DATABASE BIOSIS [Online] BIOSCIENCES INFORMATION SERVICE, PHILADELPHIA, PA, US; June 2002 (2002-06) LEE SEO-HO ET AL: "Enhancement of tissue type plasminogen activator (tPA) production from recombinant CHO cells by Low Electromagnetic Fields." Database accession no. PREV200200447597 XP002249724 & JOURNAL OF MICROBIOLOGY AND BIOTECHNOLOGY, vol. 12, no. 3, June 2002 (2002-06), pages 457-462, ISSN: 1017-7825

## Description

### Field of the Invention

The present invention relates to devices for production of thrombolytic peptides upon delivery of specified electrical currents to the targeted vascular tissue.

### Background of the Invention

Current medical practices call for diagnosing, testing and treating thrombotic events with various agents. In past years there have been great strides in the development of agents that have improved therapeutic and diagnostic application for thrombotic diseases. For example, scientists and medical researchers have produced recombinant tissue plasminogen activator as an important treatment modality in thrombolytic therapy.

There are currently two main types of vascular disease that have been found to be especially suitable for treatment by thrombolytic therapy, namely myocardial infarctions and ischemic stroke caused by interrupted or reduced blood flow. In both medical conditions, a blood clot or thrombus formation restricts the flow of blood to the tissue of the heart or the brain.

This type of blockage in the heart can cause an infarction (MI) where the flow of blood to a certain part of the myocardium or cardiac muscle is interrupted, generally resulting in a localized area of dead myocardial tissue that is surrounded by an area of myocardial tissue receiving reduced blood flow. This area of reduced blood flow is called a zone of ischemia. Ischemia in the heart is generally present in those with coronary vessel blockage which resulted in the heart attack. Other people suffer from diffuse coronary disease, which is the blockage or restricted blood flow of many coronary arteries. Re-opening the arteries to then heart is possible during the early stages of a MI by introduction of the thrombolytic agent tissue plasminogen activator (tPA). tPA improves the flow of blood to ischemic areas of the heart without resorting to by-pass surgery or efforts to reopen the blocked vessels.

Stroke is a general term for acute brain damage resulting from disease of blood vessels. Stroke can be classified into two main categories: hemorrhagic stroke (resulting from leakage of blood outside of the normal blood vessels) and ischemic stroke (cerebral ischemia due to lack of blood supply); this application is primarily concerned with the latter. The three main mechanisms of ischemic stroke are thrombosis, embolism and systemic hypoperfusion (with resultant ischemia and hypoxia). In each of these types of stroke, the area of the brain that dies as a result of the lack of blood supply thereto is also called an infarct. Obstruction of a cerebral artery resulting from a thrombus which has built up on the wall of a brain artery is generally called cerebral thrombosis. In cerebral embolism, the occlusive material blocking the cerebral artery arises downstream in the circulation (e.g., an embolus is carried to the cerebral artery from the heart). Because it is difficult to discern whether a stroke is caused by thrombosis or embolism, the term thromboembolism is used to cover both these types of stroke. When symptoms of stroke last less than 24 hours and the patient recovers completely, the patient is said to have undergone a transient ischemic attack (TIA). The symptoms of TIA are a temporary impairment of speech, vision, sensation or movement. Because a TIA is often thought to be a prelude to full-scale stroke, patients having suffered a TIA are candidates for prophylactic stroke therapy with anticoagulation agents. Thrombolytic agents, such as tissue plasminogen activator (tPA), have been used in the treatment of thromboembolic stroke. These molecules function by lysing the thrombus causing the ischemia. Such thrombolytic drugs are believed to be most useful if administered as soon as possible after the onset of an acute stroke (preferably within 3 hours) in order to at least partially restore cerebral blood flow in the ischemic region and to sustain neuronal viability. Because thrombolytic drugs may exacerbate bleeding, their use in hemorrhagic stroke is contraindicated.

In addition to MI and stoke, additional clinical complications can result from vascular disease including ischemic neuropathy. Alternatively, many patients suffer from deep vein thrombosis or superficial vein thrombosis wherein improved blood flow to the surrounding tissues would help ameliorate the condition. As previously discussed, treatment for ischemic diseases relies on improving blood flow to the ischemic area. Alternatively, when used prophylactically; treatment prevents the loss of blood flow to the tissue.

Increased levels of circulating tPA can also be used to improve therapies where the use of external medical devices could potentially cause formation of harmful blood clots. For example, formation of thrombi or clots is problematic with arteriovenous shunts (AV Shunt). Most commonly the shunts are made out of plastic tubing. The tubing is used to form an access port to dialysis machines for patients with kidney failure. During placement of the shunt it is extremely important that thrombotic clots are not formed. Higher levels of circulating tPA could help prevent thrombus formation and keep the AV shunt patent for longer term usage. Increased levels of circulating tPA could also be used in conjunction with internal medical devices. For example, mechanical heart valves can form thrombi, and use of tPA may improve their functioning:

Current therapy for reopening closed or partially closed blood vessels is performed by direct injection of tPA. During external delivery of such thrombolytic agents, large amounts are often destroyed or lost to the general circulation. This is inefficient, expensive, and can promote toxicity in certain regions. Other side effects are also possible in healthy tissue due to the inefficiency of such systemic delivery methods. There is also a need for a treatment apparatus that is cost effective and reduces the risk of side effects. There is also a need for a method and/or device that utilizes and enhances the body's natural mechanisms for thrombolysis, while avoiding the need for any introduction of foreign agents. Because the cells of the body endogenously produce tPA, a response to or anticipation of tPA would limit the occurrence of unwanted systemic hemorrhaging and bleeding.

### Summary of the Invention

The present invention provides a vascular electrode system as defined in Claim 1. The present invention may be used in an electrical stimulation apparatus for delivering an electrical field or electrical current to the vascular tissue over a predetermined period of time in order to stimulate a cell-initiated thrombolytic peptide response. The electrical stimulation apparatus has an electrical field or electrical current-generating unit (collectively EGU) including a power supply and a control mechanism interconnected with the power supply; and a plurality of electrodes designed to deliver an electrical field or electrical current to the targeted vascular tissue. The plurality of electrodes are in electrical communication with the power supply and the control mechanism controls an amplitude and a duration of a period of delivery of electrical pulses from the power supply to the respective electrodes and through the vascular tissue when the electrodes are in contact with the vascular tissue at a plurality of locations. The amplitude of the electrical field or electrical current delivered to the vascular tissue and the duration of the period of delivery are sufficient to stimulate the production of thrombolytic peptides from the vascular tissue, preferably by causing cells within the vascular tissue to increase tissue plasminogen activator (tPA) expression.

The electrical field or electrical current-generating unit may be a biphasic pulsating current delivery device and the electrical field or electrical current is generated by the biphasic pulsating current delivery device. The device is able to be designed to provide a variety of electrical stimulating waveforms to stimulate the production of thrombolytic peptides, such as tPA. It is intended that any given therapy can be repeated several times a day as needed to achieve the desired therapeutic levels of tPA in circulation. In other preferred embodiments, the control mechanism includes a computer processing unit in electronic communication with the power supply, the computer processing unit being programmed to deliver the electrical stimulation apparatus to deliver a predetermined amount of electrical current or voltage over a predetermined period of delivery to the plurality of electrodes such that the electrical stimulation apparatus can deliver such electrical current or voltage to the vascular tissue when the plurality of electrodes are in contact or in proximity with the vascular tissue.

In one embodiment, a plurality of electrodes are configured in a manner selected from the group consisting of unipolar, bipolar, and multiple electrode configurations and the apparatus is preferably designed and configured to be implantable or external to the body. In another embodiment the stimulating electrode is distal to the site of the receiving electrode. The present invention provides unique electrode designs for delivery of electrical field or electrical currents to vascular tissues. The present invention provides electrodes designed to conform to the vascular architecture with uniquely designed series of parallel electrodes affixed to a common base plate that can wraparound the vasculature.

The present invention may be used in an ex vivo method of treatment of vascular tissues by providing biologically compatible cells, preferably autologous or heterologous cells, for transplant to myocardial tissue. The transplanted cells serve to provide a new source for production of tPA upon stimulation. Optionally, any of the provided cells may be stimulated before implantation in a manner described herein to increase tPA expression, or to select for expressing cells. The transplanted cells may be chosen from a large variety of differentiated or stem cells types, but are preferably of endothelial cell origin. Muscle cells would be of mesenchymal origin, unless perhaps we believe the lining of the vasculature is our target.

The present invention has several advantages. For example, delivery of tPA can be promoted without the delivery of foreign agents, which allows the body to heal naturally and minimizes potential for side effects. Yet another advantage is that electrical energy can be applied for extended periods of time with minimal risk of killing the target cells. Another advantage is that the present invention can be used to treat deep tissues, as well as superficial tissues. Certain techniques may be either invasive, minimally invasive, or noninvasive. Furthermore, the treatment of the ischemic tissue can be targeted while exposure to healthy tissue is minimized. The described electrical stimulation therapy can be used in combination with known delivery of routes of administration tPA, wherein the use of production of tPA by stimulation can be used to provide a baseline level of tPA or as a bolus to exogenously administered tPA.

The above described features and advantages along with various other advantages and features of novelty are pointed out with particularity in the claims of the present application. However, for a better understanding of the invention, its advantages, and objects attained by its use, reference should be made to the drawings which form a further part hereof and to the accompanying descriptive matter in which there are illustrated and described preferred embodiments of the invention.

### Brief Description of the Drawings

The following drawings depict certain embodiments of the invention. They are illustrative only and do not limit the invention otherwise disclosed herein:
Figure 1A: In Vitro Cell Stimulation System.
Figure 1B: Stimulation Waveform.
Figure 2A: Normalized tPA production by Endothelial Cells in vitro at 1 Hz.
Figure 2B: Normalized tPA production by Endothelial Cells in vitro at 10 Hz.
Figure 3A: Normalized Cell Viability Following In Vitro Electrical Stimulation at 1 Hz.
Figure 3B: Normalized Cell Viability Following In Vitro Electrical Stimulation at 10 Hz.
Figure 4A: Maximized Objective Function for tPA Production and Cell Viability at 1 Hz.
Figure 4B: Maximized Objective Function for tPA Production and Cell Viability at 10 Hz.
Figure 5A: Coronary Artery Placed Stent and RF From Directly Attached Coil Lead.
Figure 5B: Coronary Artery Placed Stent and RF From External Coil.
Figure 6A: Arterio-Venous Graft Placed Stent and RF From Direct Attached Coil Lead.
Figure 6B: Arterio-Venous Graft Placed Stent and RF From External Coil.
Figure 7: Schematic for Test of In Vivo Stimulation.
Figure 8A: tPA Protein Levels From Electrically Stimulated Rabbit Arteries.
Figure 8B: tPA Activity Levels From Electrically Stimulated Rabbit Arteries
Figure 9: Schematic Output Circuit for Generation of Electrical Stimulation Pulses
Figure 10A: Output Circuit During Delivery of The Cathodic Stimulation Phase
Figure 10B: Output Circuit During Discharge Phase
Figure 11A: Cylindrical Electrode Design With Varying Contact Density
Figure 11B: Wraparound Electrode Design

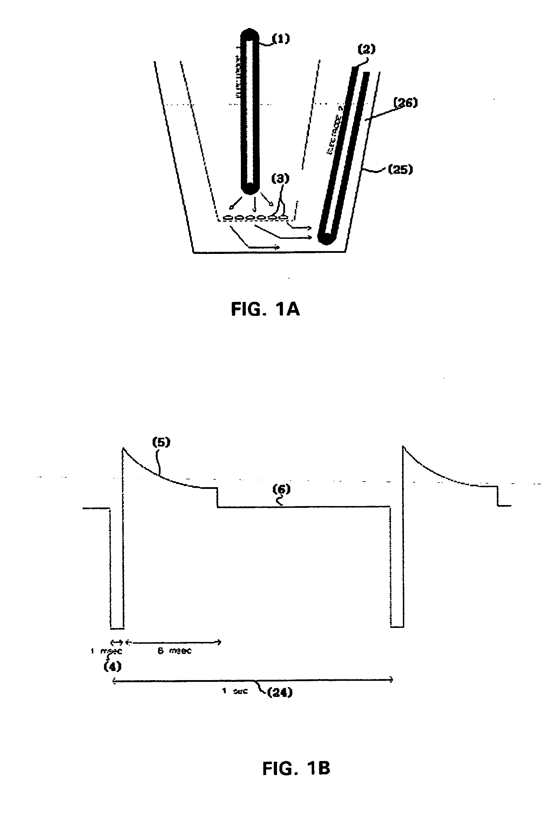

Referring now to the drawings, the electrical devices and therapies for cellular production of tPA during electrically-mediated thrombolytic peptide production are illustrated in Figures 1 through 11.

Figure IA illustrates an array of stimulated cells (3) grown in culture and placed in a testing apparatus (25) and contained in a conductive media (26) for application of various electrical field or electrical current patterns created from stimulatory electrode (1) and return electrode (2).

Figure IB illustrates one pattern of a biphasic stimulation waveform for stimulating cells or tissues for production of tPA. The biphasic waveform (24) as illustrated has an initial cathodic stimulatory phase (4) followed by the anodic stimulatory phase (5), and then a non-stimulatory phase (6).

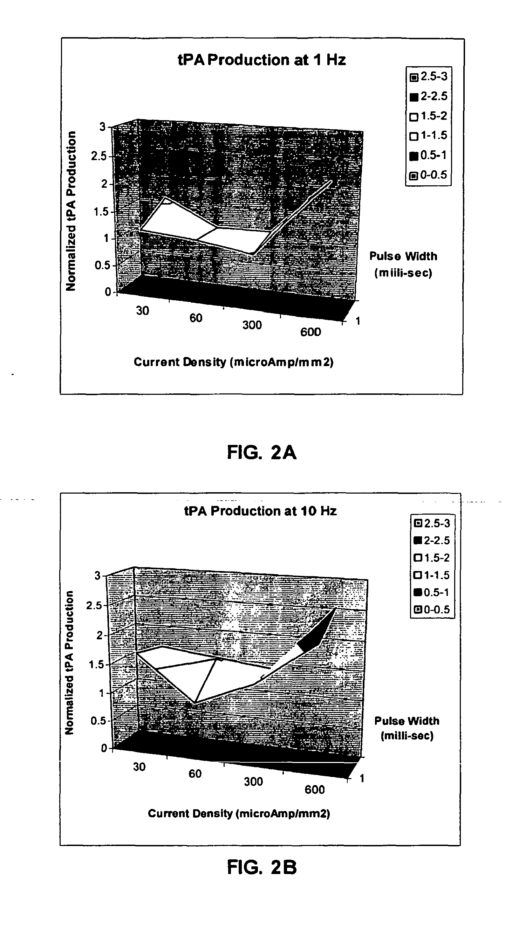

Figures 2A and 2B illustrate normalized data for tPA production by endothelial cells stimulated in vitro. Cells were stimulated at 30, 60, 300, and 600 µA/mm² with a pulse width of 1 or 10 msec and at a frequency of 1Hz (Fig. 2A) or 10 Hz (Fig.2B) using an electrical stimulation apparatus illustrated in Fig. 1A.

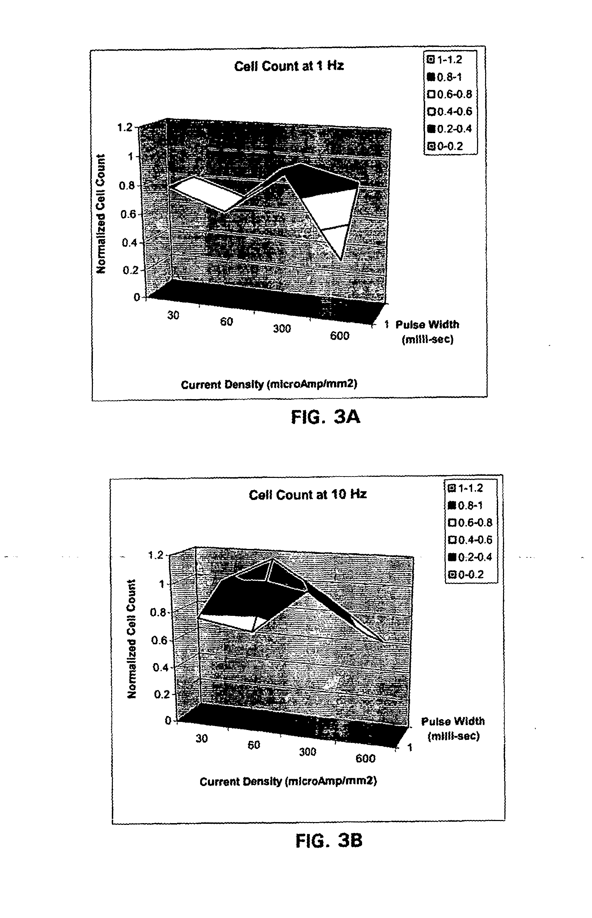

Figures 3A and 3B illustrate the corresponding normalized production of tPA per cell. Normalized production of tPA per cell maximized the electrical stimulation regime per cell in the experiment described for Figures 2A and 2B wherein endothelial cells were stimulated in vitro at 30, 60, 300, and 600 µA/mm² a pulse width at 1 and 10 msec at a frequency of 1Hz (Fig. 2A) or 10 Hz (Fig.2B) using an electrical stimulation apparatus illustrated in Fig. 1A (values for 10 millisecond stimulation are not labeled on the x-axis). The desired function predicts values where there is the minimum number of cells killed, e.g. numbers around unity are preferred.

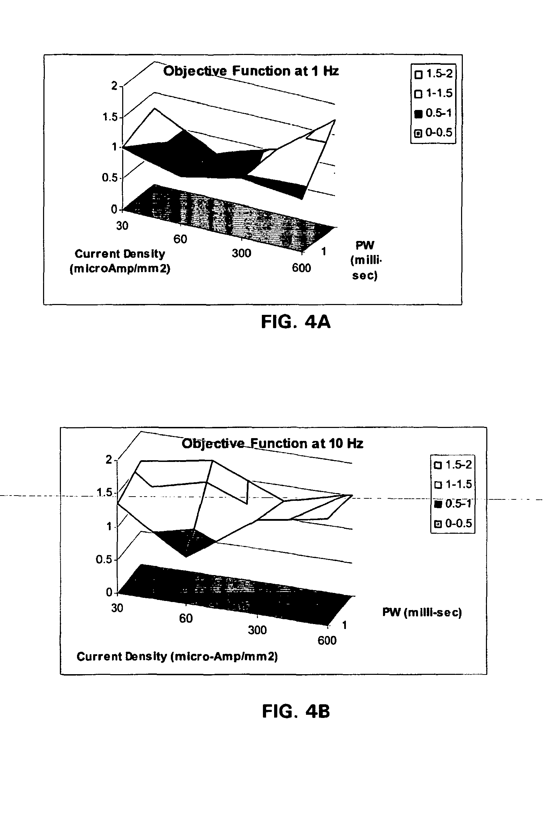

Figures 4A and 4B illustrate the objective function of maximizing the concentration of tPA and viable cells from in vitro stimulation wherein the maximized objective function is the product of the tPA concentration and the viable cell count (Maximized Objective Function = [tPA] x [viable cell count]) for stimulation at 1Hz (Fig. 4A) or at 10 Hz (Fig. 4B)) (values for 10 millisecond stimulation are not not labeled on the x-axis in either 4A or 4B).

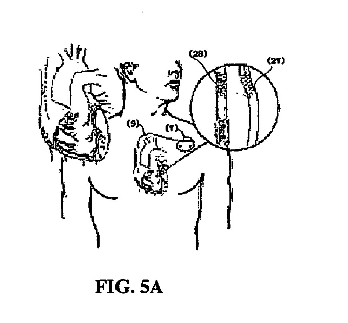

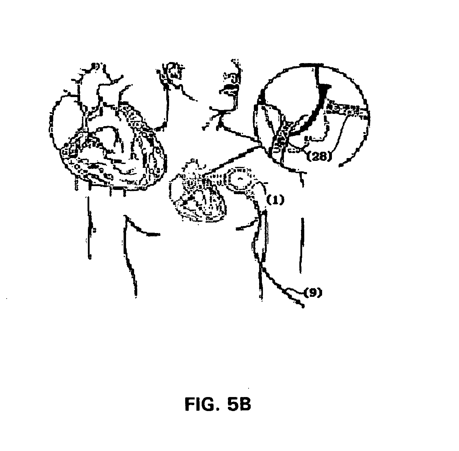

Figure 5A illustrates coronary artery disease therapy by placement of stimulating stent (27) in the coronary artery attached through venous coil lead (9) from the electrical field or electrical current generating unit (7). The electrical field or electrical current is received from the return electrode (2). Figure 5B shows alternative placement of a stimulating electrode (1) by providing an external RF coil in the body cavity to stimulate the placed return stent electrode (28) in the coronary artery (values for 10 millisecond stimulation are not labeled on the x-axis either figures 5A or 5B.).

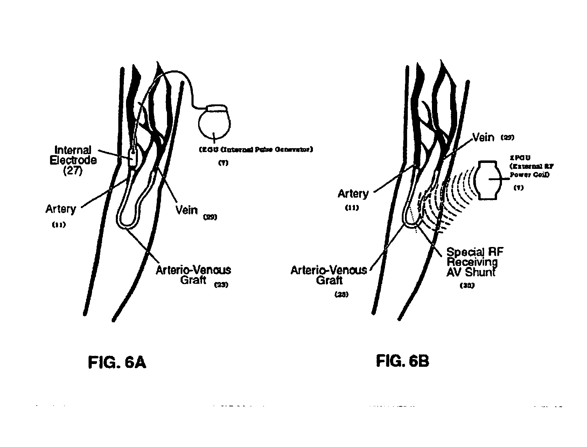

Figure 6A illustrates thrombolytic therapy by placement of stimulating stent electrode (27) in an arterio-venous graft (23) wherein the RF power is delivered by an electrically isolated coil lead directly from the electrical field or electrical current generating unit (7) which is illustrated as an implantable pulse generator (IPG). Figure 6B shows alternative place of an external RF coil as the stimulating electrode (1) which is not implanted in the body or is directly linked to placed arterio-venous receiving shunt (30) as a special receiver to the external EGU (1). Also illustrated in Figures 6A and 6B are arteries (11), veins (29), and arterio-venous graft (23).

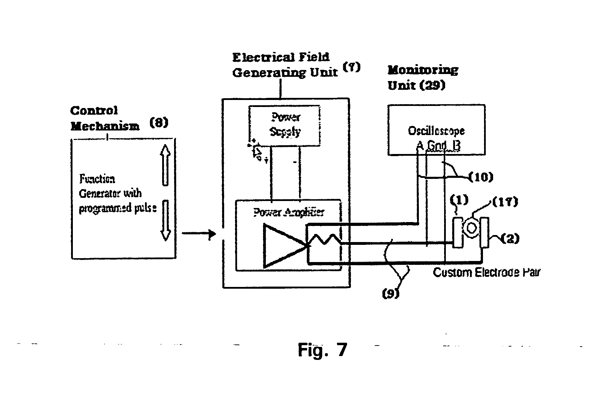

Figure 7 is a block diagram of the in vivo test apparatus for stimulating vascular tissue. Illustrated is a control mechanism (8) for programmed stimulation. The control mechanism (8) controls the current density, frequency, pulse width, and duration of therapy provided from the electrical field or electrical current generating unit (7) (illustrated here with a power supply and power amplifier). Attached to the power supply are a pair of leads (9) with stimulating electrode (1) and return electrode (2). The pair of electrodes illustrated is placed across the vascular tissue (17). Also shown is an attached oscilloscope to monitor and measure the stimulation voltage and current which serves to illustrate optional monitoring unit (29), and which, with detection leads (10), can form part of the total system.

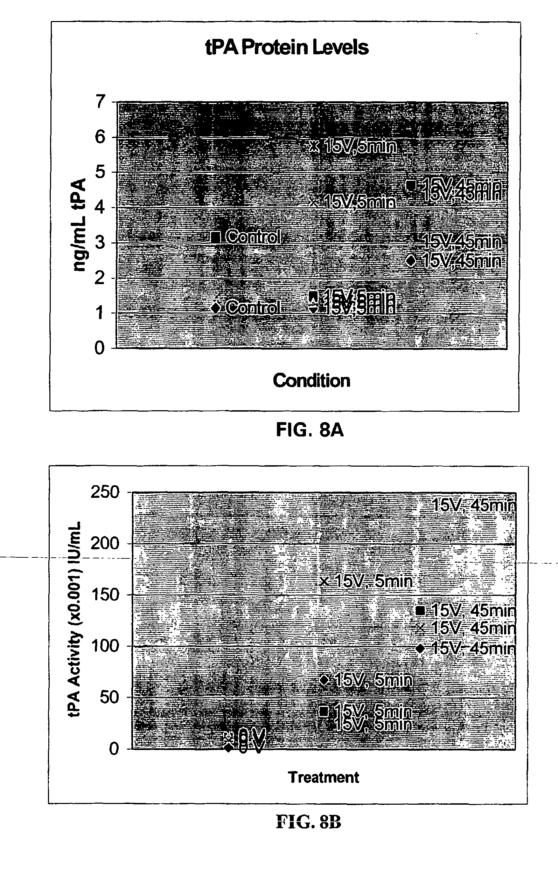

Figure 8A illustrates the measured tPA protein levels after 24 hours of stimulation at 15V for either 5 minutes or 45minutes. Figure 8B illustrates the corresponding samples measured for tPA protein activity.

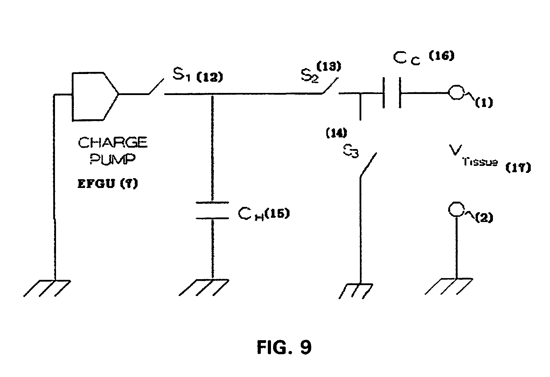

Figure 9 illustrates an output circuit for generating the electrical stimulation pulses comprising an electrical field or electrical current generating unit (7), illustrated as a charge pump, and a series of switches and capacitors for delivering the stimulus to the vascular tissue (17) through the terminal stimulating electrode (1) and return electrode (2). During off time, switches S₂ (13) and S₃ (14) are open, but S₁ (12) is closed to charge the holding capacitor, C_{H} (15). During the delivery of the cathodic stimulation, switch S₃ (14) is open, but S₂ (13) is closed to deliver the negative charge on the holding capacitor, C_{H} (15) to the tissue through the coupling capacitor Cc (16). During the delivery of the anodic stimulation, switch S₃ (14) is closed and S₂ (13) is opened. This scheme assures that the stimulus to the tissue is charge balanced, and that the anodic stimulation is obtained using the residual charge left on the coupling capacitor rather than draining power from the holding capacitor C_{H} (15).

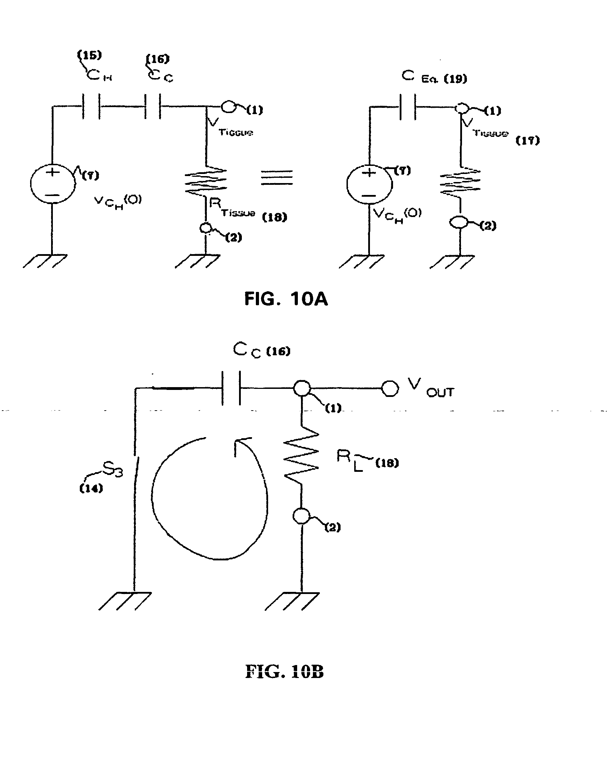

Figure 10A illustrates the delivery circuitry during the cathodic stimulation with holding capacitor C_{H} (15) and coupling capacitor C_{C} (16) connected in series when S₃ (14) is open and S₂ (13) is closed (shown as a solid line). Figure 10B illustrates the effective circuitry during the discharge phase of anodic stimulation from the electrical field or electrical current generating unit (7) where switch S₃ (14) is closed and S₂ (13) is open allowing for return of the charge from the vascular tissue (18), illustrated as R_{L} utilizing return electrode (2).

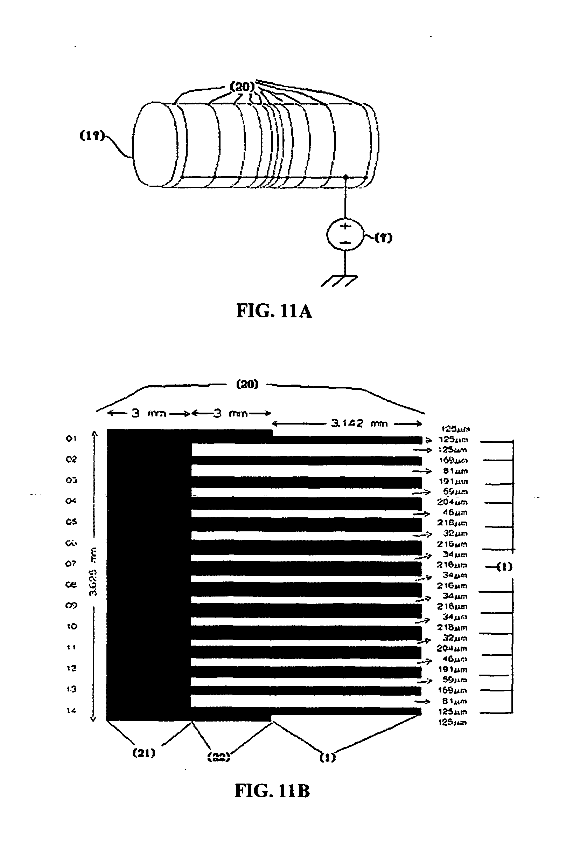

Figure 11A illustrates a cylindrical wraparound electrode(s) (20) with varying contact densities to the vasculature tissue (17). The wraparound electrode (20) is attached to electrical field or electrical current generating unit (7). Figure 11B illustrates a wraparound electrode (20) design for vascular tissue with varying electrode contact densities. The wraparound stimulating electrode (1) has base region (21) with variable contact region(s) (20). The variable contact region has variable spacing between each electrode. As presented, the contact region and the adjoining spacing region make up a constant unit dimension. The contact region of the wraparound electrode (20) is formed as part of the base region (21) with an optional middle sensing region (22) which can be used to monitor the stimulation current. In the present configuration the return electrode is placed at a distant site, forming a unipolar stimulation environment.

### Detailed Description of the Invention

Detailed description of the preferred embodiments and various other embodiments of the present invention are described below in detail with reference to the drawings, wherein like reference numerals represent like parts and assemblies throughout the several views. Reference to the various embodiments is not intended to limit the scope of the invention.

In general, the present invention relates to a vascular electrode system which may be used with an apparatus for generating an electrical field or electrical current proximal to, or within the vascular tissue, and may be used in methods of thrombolytic therapy with such an apparatus to stimulate production of a thrombolytic peptide response within cells in such body tissues. In these methods, electrical energy is delivered to cells in the targeted vascular tissue which is located in an electrical path between at least two electrodes of such an apparatus. Such delivery has been demonstrated to promote a cell-initiated thrombolytic peptides response that promotes thrombolysis and/or prevents the build up of thrombotic deposits in vascular tissue. The area of treatment in the body can be distal or proximal to the electrically stimulated vascular tissue. Because stimulated cells of the vascular tissue release thrombolytic peptides into the vascular system, they can be carried and delivered to distal sites to break down thrombi distal to the site of stimulation. Although not to be locked to any particular mechanism of action, the cell-initiated thrombolytic peptide response is believed to include a cellular process of increased production of tPA which is either initiated or accelerated following the application of electrical stimulation to vascular tissue.

The present invention may be used in an electrical stimulation apparatus for delivering an electrical field or electrical current to the vascular tissue over a predetermined period of time in order to stimulate a cell-initiated thrombolytic peptide response. The electrical stimulation apparatus has an electrical field or electrical current-generating unit including a power supply and a control mechanism interconnected with the power supply; and a plurality of electrodes designed to deliver an electrical field or electrical current to the targeted vascular tissue. The plurality of electrodes are in electrical communication with the power supply and the control mechanism controls an amplitude and a duration of a period of delivery of electrical pulses from the power supply to the respective electrodes and through the vascular tissue when the electrodes are in contact with the vascular tissue at a plurality of locations. The amplitude of the electrical field or electrical current delivered to the vascular tissue and the duration of the period of delivery are sufficient to stimulate the production of thrombolytic peptides from the vascular tissue, preferably by causing cells within the vascular tissue to increase tissue plasminogen activator (tPA) expression. For example, in reference to Figure 7, the stimulating electrode (1) and the return electrode (1) are in electrical communication via electrical lead (9) with an electrical field or electrical current generating unit (EGU) (7). The EGU is in electrical communication with control mechanism (8). In preferred embodiments, the control mechanism (8) is a computer processing unit (CPU) which is programmed to generate a preferred electrical field or electrical current within or proximal to the vascular tissue.

In an alternate embodiment of the present invention, as illustrated in Figure 9, the EGU(7) includes an electrical power supply, one or more switches as illustrated by (12),(13),(14) so that the circuit can be broken or directed to build up charge on one or more of the capacitors (15) or (16). In other embodiments the EGU (7) is a biphasic pulsating current delivery device that delivers a biphasic waveform as illustrated in Figure 1B. In order to effectively provide computer controls for the EGU, appropriate modifications are made to provide for programmed control of these devices by a CPU. In preferred embodiments, the source of current is controlled by a microprocessor or other computer processing unit (CPU) which is preferably programmed to cause the electrical stimulation apparatus to deliver a predetermined amount of electrical current over a predetermined period of delivery to the plurality of electrodes such that the electrical stimulation apparatus can deliver such electrical field or electrical current to the vascular tissue when the plurality of electrodes are in contact with the vascular tissue.

In one embodiment of the present invention, the electrical field or electrical current-generating unit is a biphasic pulsating current delivery device and the electrical field or electrical current is generated by the biphasic pulsating current delivery device. The amplitude of the electrical current delivered to the vascular tissue by the biphasic pulsating current delivery device is preferably from about 0.1 V to about 25V, and more preferably from about 5V to about 15V. As would be recognized by one skilled in the art the delivered voltages described would correspond approximately to .2 milliamps to about 50 milliamps, and more preferably from about 10 milliamps to about 30 milliamps. The duration of the period of delivery of electrical current per discharge is about 0.1 ms to about 20 ms, and more preferably about 1 ms to about 10 ms, and even more preferably about 1 ms to about 5ms. The delivered current density is about from 30 µA/mm² to 600 µA/mm². In further preferred embodiments, the electrical field or electrical current is produced by a number of pulses in the range of from 1 to about 1 million pulses with a frequency between about 0. 1 Hz to about 20 Hz, and more preferably about 1 Hz to about 10 Hz, and the duration of therapy may vary between about 0.0001 seconds to several days, and more preferably from about 1 minute to 1 day, and even more preferably about 5 minutes to about 1 hour. It is intended that any given therapy can be repeated several times a day as needed to achieve the desired therapeutic levels of tPA in circulation

The present invention provides unique designs for vascular electrodes. A number of electrodes designs are well known in the art, but few if any are designed to stimulate arteries and veins. An alternative configuration of the electrical stimulation electrode for stimulating the vasculature is shown in Figures 11A and 11B. Figure 11A illustrates a cylindrical wraparound electrode(s) (20) with varying contact densities to the vasculature tissues (17). The wraparound electrode (20) is attached to electrical field or electrical current generating unit (7). Figure 11B illustrates a wraparound electrode (20) design for vascular tissue with varying electrode contact densities. The wraparound stimulating electrode (1) has base region (21) with variable contact region(s) (20). The variable contact region has variable spacing between each electrode. As presented, the contact region and the adjoining spacing region make up a constant unit dimension. The contact region of the wraparound electrode (20) is formed as part of the base region (21) with an optional middle sensing region (22) which can be used to monitor the stimulation current. In the present configuration the return electrode is placed at a distant site, forming a unipolar stimulation environment.

A dedicated electrode system designed specifically for implantation allows for chronic administration of electric current to target vasculature tissue for purposes of stimulating thrombolytic therapy. Design of the electrode can take on a number of different shapes, and sizes, depending on the nature of the target tissue. In the case of heart muscle or other tissue, the electrode(s) can consist of a straight pin, a screw, a helix, or a patch. The patch can be further divided into mechanisms for delivery either to a smooth surface for contact with the heart, or with various barbs, hooks, needles, clamps, staples, and the like for penetration into some portion of the heart muscle.

The electrode systems used with the present invention may be unipolar or bipolar. A mono electrode system has an electrode of one polarity positioned on one structure and an electrode of an opposite polarity positioned on a different structure. In a bipolar electrode, electrodes of both polarities are mounted on a single structure such as catheter or probe and are electrically isolated from one another. Additionally, a single electrode may be used for each polarity or a group of electrodes might be used. For example, there might be two or more electrodes placed over a targeted thrombolytic area of vascular tissue where it is desired to stimulate the production of thrombolytic peptides.

In principle, any conductor, such as metal or electrically conducting organic polymer (or combination of the two), can serve as the electrode material. Additionally, the materials used to form the electrodes may be either sacrificial or nonsacrificial. Examples of sacrificial materials include silver/silver chloride, tin, iron, lithium, amalgams, and alloys thereof. Examples of nonsacrificial materials include platinum, gold, and other noble metals. The electrodes also can be formed with zirconium, iridium, titanium, platinum, certain carbons, and stainless steel, which may oxidize under certain circumstances. Alternatively, certain conductive polymers may be used. The polarity of the delivering as well as the return electrode may be in either direction as long as the circuit is closed.

Thrombolytic therapy may be provided by use of an array of electrodes with at least one electrode, having one polarity and at least one other electrode, preferably a plurality, having an opposite polarity. As can be appreciated by one skilled in the art, the electrical field or electrical current can be transferred through the tissue by RF energy ("RF" indicating transferred energy is in the radio frequency band of the electromagnetic spectrum). In certain embodiments, the stimulating pulses may be directed to more than one location, optionally selected between 2, 3, 4, 5 or more, locations.

In yet another embodiment, there is a sensing electrode separate from the stimulating (1) and return electrodes (2), respectively, which is especially useful for cardiovascular applications. In this embodiment, the sensing electrode is used to sense the electrical activity of the heart and time the delivery of the electrical energy during the refractory period. In this regard, it is noted that the heart muscle is in a state of general relaxation during a refractory period which follows the initiation of contraction of the heart muscle. In a preferred embodiment, stimulation to cause production of thrombolytic peptides from the vascular tissue are synchronized so that pulses of electrical energy are generated to deliver an electrical field or electrical current to the heart during these refractory periods in order to reduce the risk of creating an arrhythmia. In preferred embodiments, the apparatus will monitor the heart with a sensing lead so that the CPU can provide the programmed synchronization necessary to provide the appropriate timing to deliver pulses during the refractory period. In further embodiments, the sensing lead in coordination with the CPU will also have heart pacemaking capabilities to allow it to pace the heart to facilitate the synchronization of the pulsed electrical field or electrical current generation with the occurrence of the refractory period.

In one embodiment, an electrical current is delivered to the tissue. The current density to the tissue is between about 30 µA/mm² and about 600 µA/mm², preferably between about 30 µA/mm² and about 300 µA/mm², more preferably between 30 µA/mm² and about 60 µA/mm² is preferably conducted between the electrodes. In another embodiment, the amplitude of the current delivered is between about 0. 1V and about 25 V, and more preferably from about 5V to about 15V, although other current amplitudes can be used. In a further embodiment, the delivered voltages would correspond approximately to .2 milliamps to about 50 milliamps, and more preferably from about 10 milliamps to about 30 milliamps. In an embodiment that uses pulsed or alternating waveform, the amplitude of the current can be adjusted in relation to the pulse width and duty cycle, which allows control over the overall density of the current being emitted from the electrode. In a preferred embodiment a biphasic pulsating voltage is applied to the tissue. In another embodiment pulse width is in the range from about 0. 1 ms to about 100 ms, and more preferably the pulse width is 1ms to about 10ms, and even more preferably about 1ms. Frequency that these stimulation pulses are delivered could range from once a second to ten times a second, e.g., 1-10 Hz. Figures 2A, 2B, 3A, 3B, 4A, and 4B tPA illustrate the production can be optimized. Figures 3A and 3B indicate that a threshold for in vitro stimulation is a achieved for tPA production. This threshold was found to occur at 300 µA/mm² at 1 Hz and 10 Hz of stimulation. Although the desired effect is to maximize the tPA production per cell, some cell death was observed. To reduce the cell death caused by the application of the electrical stimulation, cell viability counts were done in each experiment to quantify the changes in cell population, which is shown in Figures 3A and 3B for the two stimulation patterns. The data observed for cell viability indicates there is a threshold of increased damage to the cells that starts at 300 µA/mm² at 1 Hz of stimulation and at lower intensities at 10 Hz stimulation. Taking account of both the production of tPA per cell and cell viability, an objective function of both parameters can be produced (see Figures 4A and 4B). Data from the in vitro experiments indicate that in the conditions tested that one preferred combination of stimulation could be achieved using a current density of 60 µA/mm², 10 milli-seconds, and frequency of 10Hz. These experiments show how to achieve preferred values of stimulation to maximize the production of tPA.

In addition to the in vivo and in vitro method described above, an alternative embodiment can be used with implanted cells. In this embodiment cells may be stimulated ex vivo prior to transplantation or after transplantation of the cells, or both. Cells may be of either mesenchymal, endothelial, or exodermal origin, and may be stem cells, progenitor, or a differentiated cell type. Cells may be delivered through any route of administration and in any form, e.g., in solution or on a patch. One preferred type of cells for transplantation are of endothelial origin, such as, human coronary artery endothelial cells or human umbilical vein endothelial cells. Another preferred cell type for transplantation are of mesenchymal origin, selected from the group of pluripotent stem cells, mesenchymal stem cells, or hematopoietic stem cells, or as part of a more complex implant including genetically engineered cells. It is envisioned that endothelial stem cells can be used as the transplant source. In another embodiment epithelial cells may be used to produce tPA. Cells may be selected to be autologous, allogenic, or xenogenic in origin. Cells are preferably autologous cells that have been removed form the prospective patient, which are biologically compatible with the vascular tissue. Prestimulation of cells is done in a sufficient manner to improve some function of tPA expression by the cells, wherein the amplitude of the electrical field or electrical current delivered to the vascular tissue and the duration of the period of delivery is sufficient to cause the cells to increase tPA expression. Alternatively transplanted cells are not prestimulated and are directly injected into the vascular tissue and then stimulated during or after implantation. In another preferred embodiment the cells may be genetically engineered, for example, to add additional tPA genes or modified tPA genes.

As described above, the use of electrical energy stimulates the target tissue's natural ability to heal or revascularize in an ischemic area. The delivery of electrical current generally improves blood flow. It also has been shown herein that appropriate electrical stimulation can cause increased production of tPA and blood flow in thrombotic models. In particular, passing low amperage electrical current through body tissues causes cells to increase overall expression of tissue plasminogen activator (tPA), which is believed to prevent thrombotic closure that would otherwise occur. The above concepts are demonstrated in the following examples.

### EXAMPLES

### In Vitro Stimulation of Endothelial Cells

In Vitro Cellular production of tPA was carried out using human coronary artery endothelial cells (Clonetics, San Diego, CA (cat# CC-2585) or human umbilical vein endothelial cells (Clonetics, San Diego, CA (cat# CC-2517). The endothelial cells were seeded on Falcon Cell Culture Inserts, Cat. No. 353040, at 2.5x10⁴ cells/cm² (1.05x10⁵ cells/insert). The inserts were placed in the companion plate and grown in EGM^{™}-2-Endothelial Cell Medium-2, Clonetics CC-3162, for three days prior to stimulation. Immediately prior to stimulation the cells were switched to EGM^{™}-2 - Endothelial Cell Medium-2, without fetal bovine serum, and the cell culture inserts were placed in the holding chamber and stimulated.

Figure 1A illustrates equipment used for stimulating in vitro endothelial cells. The Falcon Cell Culture Inserts were then inserted into the holding chamber containing a conductive media. An electrode is placed in the lower chamber and one in the insert. The bottom of the insert is a microporous membrane which allows media and current to pass through while cells remain in the upper chamber. The negative electrode is placed in the side well containing Endothelial Cell Medium-2, without fetal bovine serum, and the cells. The cells in the cell culture inserts were rotated through the electrical field or electrical current, 90 degrees/turn, with each quadrant of the cell culture chamber being exposed to 10 minutes of the electrical stimulation. The total time for electrical stimulation was 40 minutes per cell culture insert (4x10 minutes).

The amount of tPA protein produced was quantified using a tPA Elisa assay. After Stimulation the viable cells were quantified by physically counting just after harvesting supernatant samples, approximately 24 hours post-stimulation. Cell viability was also evaluated using the Live/Dead Viability/Cytotoxicity Kit (Molecular Probes #L-7013).

### In Vivo Stimulation Device

Stimulation electrodes consist of two parallel stainless steel plates, both 3 cm in length, attached to a plastic insulating base. The distance between the two electrodes was adjusted via two set- screws. The femoral artery, once dissected free of surrounding tissue is carefully placed between the two electrodes.

### 1. In Vivo Production of tPA with Electrical Stimulation

Studies of in vivo production of tPA after electrical stimulation were done using NZW male rabbits. The left femoral artery of each rabbit in the stimulation group was cleaned, isolated, and stimulated with 15 volts for either 5 minutes or for 45 minutes using 25 mm long electrodes placed 1.7 mm apart along the femoral arteries of each rabbit. tPA production was measured at 15 min, 24 hours, and 48 hours after stimulation.

Assays for tPA activity were performed by incubating phosphate buffered saline in the lumen of the femoral artery for 30 minutes. tPA level, activity and tPA mRNA were then measured using a tPA ELISA assay, activity levels of tPA were measured using a standard tPA enzyme activity assay measurement, and rt-PCR was used to measure tPA levels of RNA, respectively, which are summarized below:

**ELISA Data:**

| Harvest Time | Control | 15 V, 5 min | 15 V, 45 min |
|---|---|---|---|
| 15 min | | 3.496 ng/mL | |
| 24 hours | 2.155 ng/mL | 2.770 ng/mL | 3.644 ng/mL |
| 48 hours | 3.000 ng/mL | 2.921 ng/mL | 6.089 ng/mL |

**tPA Activity Data:**

| Harvest Time | Control | 15 V, 5 min | 15 V, 45 min |
|---|---|---|---|
| 15 min | | 6.13 x 10e-3IU//mL | |
| 24 hour | 8.37 x 10e-3IU/mL | 79.14 x 10e-3IU/mL | 147.2 x 10e-3IU/mL |
| 48 hours | 24.5 x 10e-3IU/mL | 22.6 x 10e-3IU/mL | 10.07 x 10e-3IU/mL |

**PCR Data**

| Voltage | Time Stimulation | Harvest |
|---|---|---|
| 15 V | 5 min | 14 hour harvest: 65% increase in tPA RNA levels over control |
| 15 V | 5 min | 48 hour harvest: 3% increase in tPA RNA levels over control |

Histology Data: Endothelial cell loss was observed in almost all samples, including controls. Some evidence of inflammation and necrosis in selected samples, including controls.

As it can be seen from Figure 8A and 8B, and the tables presented above measuring tPA protein activity and RNA message levels after application of the electrical stimulation caused a general increase in the tPA concentration in the rabbit model. This data indicates that active amounts of tPA can be produced into the circulation by providing effective electrical stimulation of the femoral artery.

### 2. Electrical Stimulation in an In Vivo Thrombosis Model

This study was performed in a rabbit thrombosis model to demonstrate the thrombolytic effect from electrical stimulation of the femoral artery. Electrical stimulation was applied to a segment of one femoral artery while the other femoral artery did not receive electrical stimulation. Twenty-four hours post stimulation; a thrombogenic implant was placed in the artery, distal to the area of the stimulation electrode in the stimulated tissue. Blood flow was monitored in both arteries for 1 hour after thrombus formation was initiated. The animal was then terminated, and both femoral arteries were analyzed pathologically and histologically.

The in vivo study in rabbits followed commonly well known sterilization and surgical procedures. The study comprised the following steps: 1) placement of the electrodes around each femoral artery; 2) electrical stimulation in the right femoral artery, sham stimulation in left femoral artery; 3) twenty-four hour return of animal; 4) initiation of thrombus formation via ethanol soaked braided silk suture implant plus a distally applied clamp in the stimulated artery; 5) monitor blood flow in both arteries for one hour; and 6) harvest vessels, sacrifice animal, and analyze the stimulated and sutured areas of the vascular tissue.

Surgery was done aseptically under anesthesia. Access to the femoral vascular compartment was done via a surgical incision in the inguinal region. The femoral artery was dissected from the associated connective tissue from the inguinal ligament to approximately 2 cm below the internal femoral bifurcation. This surgery was done bilaterally to expose both femoral arteries.

Once both femoral arteries were dissected, a vascular electrode was applied in the most proximal arterial segments while avoiding any damage to the artery. Warm saline was applied to the vessel and electrode plates to improve electrical conduction.

The electrodes were connected to an external stimulator wherein the parameters and time of stimulation consisted of 15 Volts, 1 msec cathodic, 8 msec anodic, balanced pulse, 1 Hz, for 45 minutes. The delivered voltage (Vstim) and current (Istim) were monitored. Following the completion of electrical stimulation in the right femoral artery and a sham procedure on the left femoral artery, the electrodes were retrieved and return of blood flow confirmed. The incision was then closed and the animal was allowed to recover.

The femoral arteries were re-exposed 24 hours later using sterile technique. To initate a thrombus in the previously stimulated artery, sutures were placed through the vessel. Braided silk suture was soaked in 100% EtOH prior to use to remove any thromboresistant coating (EtOH is also known to be thrombogenic) prior to use. A suture was placed passed through the artery two times such that the second stitch was perpendicular to the first, creating a thrombogenic foreign body in the lumen. In addition, a clamp was placed distal to the suture for 15 minutes to occlude flow. Once the clamp was removed blood flow in both femoral arteries was measured for the next hour at a site distal to the suture using a Transonic Systems Inc. flow probe. After completion of the blood flow measurements, areas of stimulation and suturing of both femoral arteries were harvested prior to euthanasia. Harvested tissue was fixed in formalin for tissue staining.

Results from the above study also indicated a trend showing an increase in blood flow with electrical stimulation, where the average flow in the stimulated artery was at the 60 % of the preinsult level while the unstimulated arteries had the average flow at the 39 % of the preinsult level, 24 hours after the insult.

## Claims

1. A vascular electrode system comprising a plurality of parallel stimulating electrodes (20) attached to a common base plate (21) which can be conformed to the vascular structure by wrapping said electrodes around the vasculature; wherein each electrode comprises a contact region (20) and wherein the contact regions of adjacent electrodes are separated by a spacing region; **characterized in that**
the plurality of stimulating electrodes (20) has contact regions of varying width and varying contact spacing between adjacent electrodes.

2. A vascular electrode system of claim 1, wherein the plurality of stimulating parallel electrodes comprises at least 5 electrodes attached to the common base plate.

3. A vascular electrode system of claim 1, wherein the plurality of parallel electrodes comprises at least 10 electrodes attached to the common base plate.

4. A vascular electrode system of claim 1 having the return electrode as part of the base plate.

5. A vascular electrode system of claim 1 having a return electrode member comprising at least one electrically isolated parallel electrode member attached to the base plate.

6. A vascular electrode system of claim 1 having a return electrode placed at a distant site from the plurality of stimulating electrode members.

7. A vascular electrode system of any preceding claim, which produces a uniform current density with minimal variation in the longitudinal axis.

8. A vascular electrode system of claim 1 wherein the current is evenly distributed in the radial direction across the stimulating electrodes.

9. A vascular electrode system of claim 1 wherein the plurality of stimulating electrodes are spaced to deliver current evenly in the radial direction to the vascular tissue.

## Patentansprüche

1. Gefäßelektrodensystem mit einer Anzahl an eine gemeinsame Basisplatte (21) angebrachter paralleler Stimulationselektroden (20), welches entsprechend der Gefäßstruktur geformt werden kann, indem die Elektroden um das Gefäß gewunden werden; wobei jede Elektrode einen Kontaktbereich (20) aufweist, und wobei die Kontaktbereiche benachbarter Elektroden durch einen Zwischenraumbereich getrennt sind; **dadurch gekennzeichnet, dass**
die Anzahl von Stimulationselektroden (20) Kontaktbereiche variabler Breite und variablen Kontaktzwischenraum zwischen benachbarten Elektroden aufweist bzw. aufweisen.

2. Gefäßelektrodensystem nach Anspruch 1, bei dem die Anzahl paralleler Stimulationselektroden wenigstens fünf Elektroden umfasst, die an die gemeinsame Basisplatte angebracht sind.

3. Gefäßelektrodensystem nach Anspruch 1, bei dem die Anzahl paralleler Elektroden wenigstens zehn Elektroden umfasst, die an die gemeinsame Basisplatte angebracht sind.

4. Gefäßelektrodensystem nach Anspruch 1 mit einer bzw. der Rückkehr- bzw. Rückleitungselektrode als Teil der Basisplatte.

5. Gefäßelektrodensystem nach Anspruch 1 mit einem Rückleitungselektrodenelement mit wenigstens einem elektrisch isolierten, parallelen Elektrodenelement, das an die Basisplatte angebracht ist.

6. Gefäßelektrodensystem nach Anspruch 1 mit einer Rückleitungselektrode, die an einem Ort in einem Abstand zu der Anzahl von Stimulationselektrodenelementen platziert ist.

7. Gefäßelektrodensystem nach einem der vorstehenden Ansprüche, das eine gleichmäßige Stromdichte mit minimaler Variation in der Längsachse produziert.

8. Gefäßelektrodensystem nach Anspruch 1, bei dem der Strom in der radialen Richtung gleichmäßig über die Stimulationselektroden verteilt ist.

9. Gefäßelektrodensystem nach Anspruch 1, bei dem die Anzahl von Stimulationselektroden beabstandet sind, um Strom in der radialen Richtung gleichmäßig an das Gefäßgewebe abzugeben.

## Revendications

1. Système d'électrodes vasculaires comportant une pluralité d'électrodes de stimulation parallèles (20) reliées à une plaque de base commune (21) qui peut être adapté à la structure vasculaire en enroulant lesdites électrodes autour du système vasculaire ; dans lequel chaque électrode comporte une région de contact (20) et dans lequel les régions de contact d'électrodes adjacentes sont séparées par une région d'espacement ; **caractérisé en ce que** la pluralité d'électrodes de stimulation (20) a des régions de contact à largeur variable et un espacement de contact variable entre des électrodes adjacentes.

2. Système d'électrodes vasculaires selon la revendication 1, dans lequel la pluralité d'électrodes de stimulation parallèles comporte au moins 5 électrodes reliées à la plaque de base commune.

3. Système d'électrodes vasculaires selon la revendication 1, dans lequel la pluralité d'électrodes parallèles comporte au moins 10 électrodes reliées à la plaque de base commune.

4. Système d'électrodes vasculaires selon la revendication 1, ayant l'électrode de retour en tant que partie de la plaque de base.

5. Système d'électrodes vasculaires selon la revendication 1, ayant un élément d'électrode de retour comportant au moins un élément d'électrode parallèle électriquement isolé relié à la plaque de base.

6. Système d'électrodes vasculaires selon la revendication 1, ayant une électrode de retour placée sur un site distant de la pluralité d'éléments d'électrodes de stimulation.

7. Système d'électrodes vasculaires selon l'une quelconque des revendications précédentes, qui produit une densité de courant uniforme avec une variation minimale dans l'axe longitudinal.

8. Système d'électrodes vasculaires selon la revendication 1, dans lequel le courant est uniformément réparti dans la direction radiale à travers les électrodes de stimulation.

9. Système d'électrodes vasculaires selon la revendication 1, dans lequel la pluralité d'électrodes de stimulation est espacée afin de délivrer un courant de manière régulière dans la direction radiale au tissu vasculaire.
